# EUROPEAN PATENT APPLICATION

(11) **EP 3 738 536 A1**
(43) Date of publication of application: **18.11.2020**
(21) Application number: 20175093.2
(22) Date of filing: 15.05.2020
(51) Int. Cl.: A61B 18/14, A61B 18/00, A61B 18/12, A61B 18/18

(54) **MONOPOLAR ELECTRODES FOR USE IN ELECTROSURGERY**

(30) Priority: 17.05.2019 US 201916415412
(71) Applicant: Covidien LP, Mansfield, MA 02048 (US)
(72) Inventor: ALLEN, James, D, Broomfield, CO 80020 (US)
(74) Representative: Maschio, Antonio

(57) **Abstract**

An electrosurgical instrument includes a shaft, an elongated electrosurgical blade coupled to the shaft, and a housing. The shaft is coupled to a source of electrical energy and the electrosurgical blade is coupled to the shaft. The electrosurgical blade defines a longitudinally-extending crease and is configured to be manually bent.

## Description

### FIELD

This disclosure relates to a hand-held electrosurgical instrument including a monopolar electrode for cutting tissue.

### BACKGROUND

Electrosurgery involves the application of high-frequency electric current to cut or modify biological tissue during an electrosurgical operation. The two main modes of electrosurgery are monopolar and bipolar electrosurgery. Monopolar electrosurgery is commonly used due to its versatility and effectiveness. Electrosurgical instruments are typically hand-held instruments, e.g., electrosurgical pencils that transfer electrosurgical energy to a tissue site via an electrosurgical electrode. To affect cutting of body tissue, the electrode is typically a thin electrode with a small contact area. The shape and size of the electrode may vary depending on the purpose of the surgery.

### SUMMARY

In accordance with an aspect of the disclosure, an electrosurgical instrument is provided and includes a shaft, an elongated electrosurgical blade coupled to the shaft, and a housing. The shaft is configured to be coupled to a source of electrical energy. The electrosurgical blade is coupled to the shaft and is fabricated from a conductive material configured to be manually bent. The electrosurgical blade defines a longitudinally-extending crease. The housing at least partially covers the shaft and the electrosurgical blade.

In aspects, the electrosurgical blade may have a thickness from about 0.002 inches to about 0.007 inches. The electrosurgical blade may further include a grain structure oriented lengthwise.

In aspects, the housing may be overmolded about a proximal end portion of the electrosurgical blade and a distal end portion of the shaft. The housing may further define a slit in a distally-facing surface of a distal end portion thereof. The electrosurgical blade extends out of the slit.

In aspects, the housing may have a non-circular outer surface configuration to prevent rotation of the housing within a handle.

In aspects, the electrosurgical blade may have a proximal end portion welded to a distal end portion of the shaft. The electrosurgical blade may further include a first longitudinal section and a second longitudinal section angled relative to the first longitudinal section about the longitudinally-extending crease.

In aspects, the angle between the first and second longitudinal sections may be from about 120 degrees to about 175 degrees. In other aspects, the angle between the first and second longitudinal sections may be an obtuse angle that narrows in a proximal direction along the electrosurgical blade. The longitudinally-extending crease may be non-linear, linear, or curved.

In accordance with another aspect of the disclosure, an electrosurgical instrument is provided and includes an elongated shaft, an electrosurgical blade, and a housing. The elongated shaft is fabricated from a conductive material and is configured to be electrically coupled to a source of electrosurgical energy. The electrosurgical blade has a proximal end portion fixed to a distal end portion of the shaft. The electrosurgical blade may be fabricated from tungsten and defines a longitudinally-extending crease that extends along a length of the electrosurgical blade. The housing is overmolded around the distal end portion of the elongated shaft and the proximal end portion of the electrosurgical blade.

### BRIEF DESCRIPTION OF THE DRAWINGS

The above and other aspects, features, and advantages of the present disclosure will become more apparent in light of the following detailed description when taken in conjunction with the accompanying drawings, in which:
FIG. 1 is a schematic illustration of an electrosurgical system according to the disclosure;
FIG. 2 is a perspective view illustrating an electrosurgical instrument of the system of FIG. 1;
FIG. 3 is a side, perspective view illustrating an electrode assembly of the electrosurgical instrument of FIG. 2;
FIG. 4 is a side, transparent view illustrating the electrode assembly of FIG. 3; and

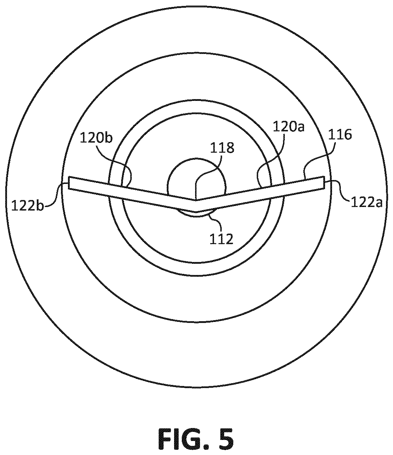

FIG. 5 is front view illustrating a distal end portion of an electrosurgical blade of the electrode assembly of FIG. 3.

### DETAILED DESCRIPTION

Particular embodiments of the disclosure are described hereinbelow with reference to the accompanying drawings. In the following description, well-known functions or constructions are not described in detail to avoid obscuring the present disclosure in unnecessary detail. As used herein, the term "distal" refers to that portion which is further from the user while the term "proximal" refers to that portion which is closer to the user or surgeon.

The following aspects of electrosurgical instrument, and in particular, electrosurgical blade electrodes for electrosurgical instruments, incorporate features to enable fast fine or aggressive precision dissection while maintaining tenacity and preventing erosion of the electrosurgical blade electrode. Aspects of the electrosurgical blade electrode disclosed herein include structural features that enable rapid dissection of tissue, precision dissection of tissue, and continuous flexibility, while maintaining rigidity during aggressive dissection of tissue.

Although the following disclosure describes electrosurgical blade electrodes as being used with a handheld pencil-type electrosurgical instrument, it is understood that the benefits of the structural features of all of the aspects of the electrosurgical blade electrodes disclosed herein may be realized by robotic surgical systems, and the following disclosure is not intended to be limiting.

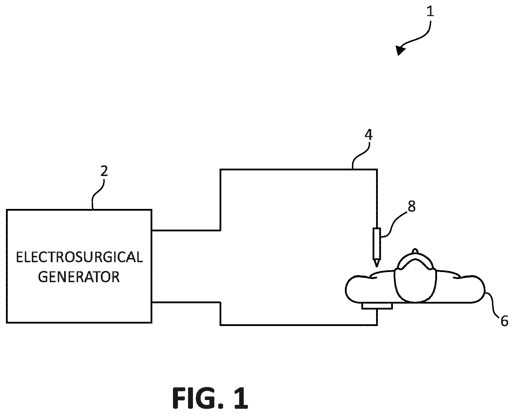

FIG. 1 illustrates an electrosurgical system 1 according to the disclosure. The electrosurgical system 1 includes an electrosurgical energy source, such as, for example, an electrosurgical generator 2, and an electrosurgical instrument 8 coupled to the generator 2. The electrosurgical instrument 8 has one or more active electrodes (not explicitly shown) for treating tissue of a patient 6. The electrosurgical instrument 8 is a monopolar instrument including one or more active electrodes, such as, for example, an electrosurgical cutting probe, ablation electrode(s), and/or the like. Electrosurgical radio-frequency (RF) energy is supplied to the electrosurgical instrument 8 by the generator 2 via a cable 4, which is connected to an active output terminal, allowing the electrosurgical instrument 8 to coagulate, ablate and/or otherwise treat tissue. Although the generator 2 is described herein as delivering RF energy, this is by example only and should not be construed as limiting. The generator 2 in various embodiments may additionally or alternatively deliver any suitable type of energy, such as ultrasonic energy, microwave energy, energy of other portions on the electromagnetic spectrum, and/or the like. In some aspects, the electrosurgical instrument 8 may be alternately configured as a bipolar instrument.

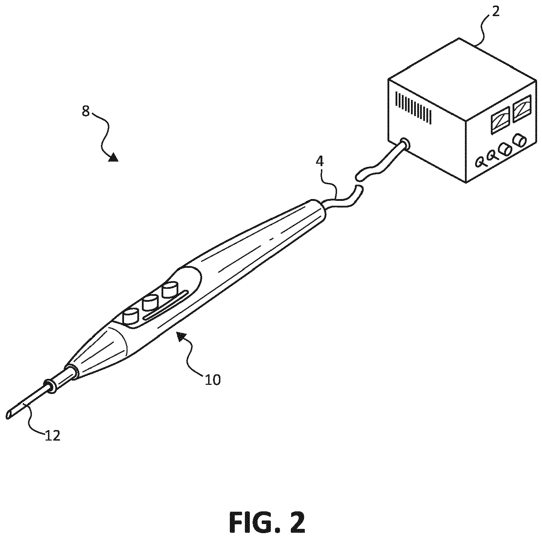

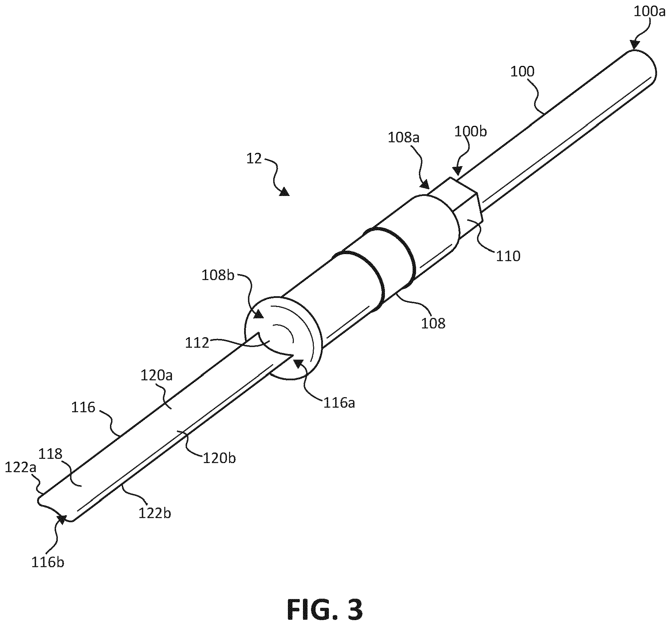

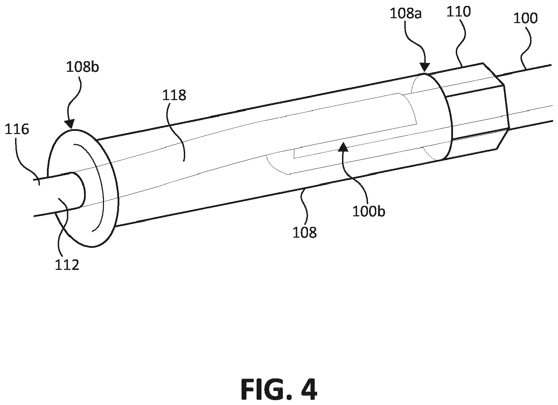

Referring to FIGS. 2-4, the electrosurgical instrument 8 generally includes a handle portion 10 and an electrode assembly 12 supported in the handle portion 10. The handle portion 10 has one or more user input buttons for activating the generator 2 via the cable 4. The electrode assembly 12 of the electrosurgical instrument 8 has a shaft 100, a housing 108, and an elongated electrosurgical blade 116. The shaft 100 includes a proximal end portion 100a and a distal end portion 100b. The proximal end portion 100a of the shaft 100 is configured to be coupled to the cable 4 via the handle portion 10 of the electrosurgical instrument 8 for receiving electrical energy from the generator 2. The shaft 100 may be fabricated from a conductive material, such as, for example, stainless steel. Other materials of construction for the shaft 100 are also contemplated.

The housing 108 of the electrode assembly 12 at least partially covers the distal end portion 100b of the shaft 100 and the proximal end portion 116a of the electrosurgical blade 116. The housing 108 may be overmolded over the shaft 100 and the electrosurgical blade 116 to assist in securing the shaft 100 and the electrosurgical blade 116 to one another. The housing 108 has a non-circular outer surface 110 on at least a proximal end portion 108a thereof configured to engage a correspondingly shaped recess (not explicitly shown) in the handle portion 10 of the electrosurgical instrument 8. For example, the housing 108 may have a proximal end 108a with a hexagonal-shaped outer surface 110. As such, upon the handle portion 10 receiving the housing 108 of the electrode assembly 12, the hexagonal outer surface 110 of the housing 108 resists rotation of the electrode assembly 12 within and relative to the handle portion 10. The housing 108 has a distal end portion 108b defining a slit 112 in a distally-facing surface thereof. The electrosurgical blade 116 is configured to extend distally out of the slit 112 of the housing 108 to provide support for the electrosurgical blade 116. In aspects of the disclosure, the outer surface 110 of the proximal end 108a of the housing 108 may be rectangular-shaped or any other shape suitable to resist rotation of the electrode assembly 12 within and relative to the handle portion 10.

The electrosurgical blade 116 of the electrode assembly 12 includes a proximal end portion 116a coupled to the distal end portion 100b of the shaft 100, and a distal end portion 116b. Coupling of the electrosurgical blade 116 and the shaft 100 may be achieved by, for example, laser welding. In aspects, the distal end portion 100b of the shaft 100 and the proximal end portion 116a of the electrosurgical blade 116 may be coupled to one another via a conductive adhesive, a fastener, or the like. The electrosurgical blade 116 is fabricated from a conductive material configured to be manually bent, such as, for example, tungsten. In aspects, the conductive material may be aluminum, steel, or any other suitable metal. The electrosurgical blade 116 may be bent several times to a preferred angle, and aggressively used, without breaking the electrosurgical blade 116.

The conductive material of the electrosurgical blade 116 includes a grain structure oriented in a particular direction for improved flexibility. For example, the grain structure of the conductive material may extend in a lengthwise manner or be angled in any suitable direction. The grain structure may be oriented in a particular direction for other desirable mechanical properties, such as, for example, improved stiffness.

The electrosurgical blade 116 defines a longitudinally-extending crease 118, such as, for example, a bend, configured to stiffen the electrosurgical blade 116. The stiffening of the electrosurgical blade 116, due to the crease 118, allows for flexibility and aggressive use of the electrosurgical blade 116. In other aspects, the electrosurgical blade 116 may define two or more longitudinally-extending creases. The crease 118 of the electrosurgical blade 116 is linear and divides the electrosurgical blade 116 into a first longitudinal section 120a and a second longitudinal section 120b disposed on each side of the longitudinally-extending crease 118. In aspects of the disclosure, the crease may be non-linear, curved, or assume any other suitable shape along the longitudinal axis of the electrosurgical blade 116.

The first and second longitudinal sections 120a, 120b of the electrosurgical blade 116 are angled relative to one another about the crease 118 from about 120 degrees to about 175 degrees. In some aspects, the angle between the first and second longitudinal sections 120a, 120b may be about 160 degrees. The angle defined between the first and second longitudinal sections 120a, 120b provides desirable mechanical properties, such as, for example, improved energy concentration at outermost, lateral edges 122a, 122b of the electrosurgical blade 116. In other aspects, the angle between the first and second longitudinal sections 120a, 120b may narrow in a proximal direction along the electrosurgical blade 116. The lateral edges 122a, 122b of the respective first and second longitudinal sections 120a, 120b are configured to concentrate energy therealong, thereby requiring less power to cut tissue.

The electrosurgical blade 116 has a set thickness of from about 0.002 inches to about 0.007 inches, and in some aspects the electrosurgical blade has a set thickness of about 0.005 inches. In aspects, the longitudinal sections 120a, 120b may have a thickness that tapers in a direction from the crease 118 outwardly toward the lateral edges 122a, 122b.

In operation, the electrode assembly 12 is inserted into the handle portion 10 of the electrosurgical instrument 8, whereupon the shaft 100 of the electrode assembly 12 is coupled to the generator 2 via the cable 4. Electrosurgical RF energy is supplied by the generator 2 to the electrode assembly 12 of the electrosurgical instrument 8 via the cable 4. More specifically, electrosurgical RF energy is transmitted from the shaft 100 of the electrode assembly 12 to the electrosurgical blade 116 of the electrode assembly 12 via the coupling of the electrosurgical blade 116 and the shaft 100.

Electrosurgical RF energy transmitted to the electrosurgical blade 116 is concentrated away from the crease 118 of the electrosurgical blade 116 towards the lateral edges 122a, 122b of the electrosurgical blade 116. One or both of the lateral edges 122a or 122b of the electrosurgical blade 116 is applied to the tissue of patient 6 at a lighter pressure than previously needed for existing active electrodes, enabling fine dissection of tissue with aggressive use of the electrosurgical blade 116. Relative to existing active electrodes, the lateral edge 122a or 122b of the electrosurgical blade 116 cuts the tissue of patient 6 quicker, smoother and with less drag or snag as it transects. Prior to or during use, a threshold force may be applied to the electrosurgical blade 116 to bend the electrosurgical blade 116 at a selected location along the length of the electrosurgical blade 116. Manually bending the electrosurgical blade 116, without resulting in breakage, allows a clinician to adjust the shape of the electrosurgical blade 116 to better suit the surgical application.

Any or all portions of any of the electrosurgical blade electrodes disclosed herein may be formed by any suitable techniques, e.g., machining techniques. For example, any cutouts, edging, ramping, or other surface geometry may be formed by known milling techniques, etching techniques, or other techniques not specifically described.

From the foregoing and with reference to the various figures, those skilled in the art will appreciate that certain modifications can also be made to the present disclosure without departing from the scope of the same. For example, electrosurgical blade electrode may include other geometrical configurations.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments.

The invention may be described by reference to the following numbered paragraphs:-:
1. An electrosurgical instrument, comprising:
   a shaft configured to be coupled to a source of electrical energy;
   an elongated electrosurgical blade coupled to the shaft and defining a longitudinally-extending crease, the electrosurgical blade fabricated from a conductive material configured to be manually bent; and
   a housing at least partially covering the shaft and the electrosurgical blade.
2. The electrosurgical instrument of paragraph 1, wherein the conductive material is tungsten.
3. The electrosurgical instrument of paragraph 1, wherein the electrosurgical blade has a thickness of from about 0.002 inches to about 0.007 inches.
4. The electrosurgical instrument of paragraph 3, wherein the thickness of the electrosurgical blade is about 0.005 inches.
5. The electrosurgical instrument of paragraph 1, wherein the housing is overmolded about a proximal end portion of the electrosurgical blade and a distal end portion of the shaft.
6. The electrosurgical instrument of paragraph 1, wherein the housing has a non-circular outer surface configuration to prevent rotation of the housing within a handle.
7. The electrosurgical instrument of paragraph 1, wherein the housing has a slit defined in a distally-facing surface of a distal end portion thereof, the electrosurgical blade extending out of the slit.
8. The electrosurgical instrument of paragraph 1, wherein the electrosurgical blade has a proximal end portion welded to a distal end portion of the shaft.
9. The electrosurgical instrument of paragraph 1, wherein the electrosurgical blade includes:
   a first longitudinal section; and
   a second longitudinal section angled relative to the first longitudinal section about the longitudinally-extending crease.
10. The electrosurgical instrument of paragraph 9, wherein the angle between the first and second longitudinal sections is from about 120 degrees to about 175 degrees.
11. The electrosurgical instrument of paragraph 10, wherein the angle between the first and second longitudinal sections is about 160 degrees.
12. The electrosurgical instrument of paragraph 9, wherein the angle between the first and second longitudinal sections is an obtuse angle that narrows in a proximal direction along the electrosurgical blade.
13. The electrosurgical instrument of paragraph 1, wherein the longitudinally-extending crease is non-linear, linear, or curved.
14. The electrosurgical instrument of paragraph 1, wherein the conductive material has a grain structure oriented lengthwise.
15. An electrosurgical instrument, comprising:
   an elongated shaft fabricated from a conductive material and configured to be electrically coupled to a source of electrosurgical energy;
   an electrosurgical blade having a proximal end portion fixed to a distal end portion of the shaft, the electrosurgical blade fabricated from tungsten and defining a longitudinally-extending crease that extends along a length of the electrosurgical blade; and
   a housing overmolded around the distal end portion of the elongated shaft and the proximal end portion of the electrosurgical blade.
16. The electrosurgical instrument of paragraph 15, wherein the electrosurgical blade has a thickness of about 0.005 inches.
17. The electrosurgical instrument of paragraph 15, wherein the housing has a hexagonal outer surface configuration to prevent rotation of the housing within a handle.
18. The electrosurgical instrument of paragraph 15, wherein the housing has a slit defined in a distally-facing surface of a distal end portion thereof, the electrosurgical blade extending out of the slit.
19. The electrosurgical instrument of paragraph 15, wherein the electrosurgical blade includes:
   a first longitudinal section; and
   a second longitudinal section angled relative to the first longitudinal section about the longitudinally-extending crease.
20. The electrosurgical instrument of paragraph 19, wherein the angle between the first and second longitudinal sections is an obtuse angle that narrows in a proximal direction along the electrosurgical blade.

## Claims

1. An electrosurgical instrument, comprising:
a shaft configured to be coupled to a source of electrical energy;
an elongated electrosurgical blade coupled to the shaft and defining a longitudinally-extending crease, the electrosurgical blade fabricated from a conductive material configured to be manually bent; and
a housing at least partially covering the shaft and the electrosurgical blade.

2. The electrosurgical instrument of claim 1, wherein the conductive material is tungsten.

3. The electrosurgical instrument of claim 1 or 2, wherein the electrosurgical blade has a thickness of from about 0.002 inches to about 0.007 inches, preferably, wherein the thickness of the electrosurgical blade is about 0.005 inches.

4. The electrosurgical instrument of any preceding claim, wherein the housing is overmolded about a proximal end portion of the electrosurgical blade and a distal end portion of the shaft.

5. The electrosurgical instrument of any preceding claim, wherein the housing has a non-circular outer surface configuration to prevent rotation of the housing within a handle.

6. The electrosurgical instrument of any preceding claim, wherein the housing has a slit defined in a distally-facing surface of a distal end portion thereof, the electrosurgical blade extending out of the slit.

7. The electrosurgical instrument of any preceding claim, wherein the electrosurgical blade has a proximal end portion welded to a distal end portion of the shaft.

8. The electrosurgical instrument of any preceding claim, wherein the electrosurgical blade includes:
a first longitudinal section; and
a second longitudinal section angled relative to the first longitudinal section about the longitudinally-extending crease, preferably wherein the angle between the first and second longitudinal sections is an obtuse angle that narrows in a proximal direction along the electrosurgical blade, further preferably, wherein the angle between the first and second longitudinal sections is from about 120 degrees to about 175 degrees, yet further preferably, wherein the angle between the first and second longitudinal sections is about 160 degrees.

9. The electrosurgical instrument of any preceding claim, wherein the longitudinally-extending crease is non-linear, linear, or curved.

10. The electrosurgical instrument of any preceding claim, wherein the conductive material has a grain structure oriented lengthwise.

11. An electrosurgical instrument, comprising:
an elongated shaft fabricated from a conductive material and configured to be electrically coupled to a source of electrosurgical energy;
an electrosurgical blade having a proximal end portion fixed to a distal end portion of the shaft, the electrosurgical blade fabricated from tungsten and defining a longitudinally-extending crease that extends along a length of the electrosurgical blade; and
a housing overmolded around the distal end portion of the elongated shaft and the proximal end portion of the electrosurgical blade.

12. The electrosurgical instrument of claim 11, wherein the electrosurgical blade has a thickness of about 0.005 inches.

13. The electrosurgical instrument of claim 11 or 12, wherein the housing has a hexagonal outer surface configuration to prevent rotation of the housing within a handle.

14. The electrosurgical instrument of claim 11, 12 or 13, wherein the housing has a slit defined in a distally-facing surface of a distal end portion thereof, the electrosurgical blade extending out of the slit.

15. The electrosurgical instrument of claim 11,12, 13 or 14, wherein the electrosurgical blade includes:
a first longitudinal section; and
a second longitudinal section angled relative to the first longitudinal section about the longitudinally-extending crease, preferably, wherein the angle between the first and second longitudinal sections is an obtuse angle that narrows in a proximal direction along the electrosurgical blade.
